# EUROPEAN PATENT APPLICATION

(11) **EP 3 896 165 A1**
(43) Date of publication of application: **20.10.2021**
(21) Application number: 19895465.3
(22) Date of filing: 05.12.2019
(51) Int. Cl.: C12N 15/77, C12N 9/10, C12P 13/00

(54) **MUTANT STRAIN HAVING ENHANCED L-GLUTAMIC ACID PRODUCING ABILITY, AND L-GLUTAMIC ACID PREPARATION METHOD USING SAME**

(30) Priority: 13.12.2018 KR 20180161190
(71) Applicant: Daesang Corporation, Seoul 02586 (KR)
(72) Inventor: KIM, Hyun Young, Gyeonggi-do 16867 (KR); LEE, Sun Hee, Gyeonggi-do 16923 (KR); KIM, Hyun Ho, Seoul 05236 (KR); JO, Young Il, Seoul 04995 (KR); YOUN, Hyung Seop, Seoul 07958 (KR); KIM, Suok Su, Gyeonggi-do 16825 (KR)
(74) Representative: Nony
(86) International application number: PCT/KR2019/017124
(87) International publication number: WO 2020/122505

(57) **Abstract**

The present disclosure relates to a mutant strain having enhanced L-glutamic acid productivity and a method of producing L-glutamic acid using the same. The mutant strain according to one embodiment of the present disclosure has reduced production of citramalate as a by-product due to weakening or inactivation of the activity of citramalate synthase and has excellent L-glutamic acid productivity. The strain having an additional mutation in the YggB protein may produce L-glutamic acid in an improved yield due to enhancement of glutamic acid release. Thus, when the mutant strain is used, it is possible to more effectively produce L-glutamic acid.

## Description

### TECHNICAL FIELD

The present disclosure relates to a mutant strain having enhanced L-glutamic acid productivity and a method of producing L-glutamic acid using the same, and more particularly, to a *Corynebacterium sp.* mutant strain having reduced production of citramalate as a by-product due to weakening or inactivation of the activity of citramalate synthase and having increased L-glutamic acid productivity, and a method of producing L-glutamic acid using the same.

### BACKGROUND ART

L-glutamic acid is a typical amino acid that is produced by microbial fermentation. Monosodium L-glutamate (MSG) may increase the preference of foods such as meat, fish, chicken, vegetables, sauces, soups and seasonings by balancing and harmonizing the overall taste of the food, may enhance the taste of low-salt foods having a salt content reduced up to 30%, and thus is widely used as a household seasoning and a seasoning for the production of processed food.

L-glutamic acid is usually produced by fermentation using mainly *Brevibacterium* sp. or *Corynebacterium* sp. strains and mutant strains thereof. In order to increase the production of L-glutamic acid by microbial culture, there has been used a method of increasing the copy number of the gene of interest to increase the expression of the gene in the L-glutamic acid biosynthesis pathway or a method of regulating the activity of the enzyme in the biosynthesis pathway by modifying the promoter of the gene. Specifically, it was disclosed that L-glutamic acid production was increased by amplifying a specific gene such as *pyc* gene or *fasR* (US Patent No. 6,852,516) or manipulating the promoter region of each of *gdh, gltA, icd, pdh* and *argG* genes (US Patent No. 6,962,805).

Citramalate synthase *(cimA)* is an enzyme that synthesizes citramalate using acetyl-CoA and pyruvate as substrates. It is known that the *cimA* gene does not exist in *Corynebacterium glutamicum,* and the *leuA* gene having high homology to the *cimA* gene plays the role of citramalate synthase in *Corynebacterium glutamicum.*

Since acetyl-CoA is used as a substrate in the fermentation pathway of L-glutamic acid, the present inventors have attempted to weaken or inactivate the activity of citramalate synthase to save the substrate acetyl-CoA, thereby further increasing L-glutamic acid productivity.

Meanwhile, it has been reported that the yggB gene of coryneform bacteria is a homologue of the yggB gene of *Escherichia coli* (FEMS Microbiol Lett., 2003, 218(2), pp. 305-309, and Mol Microbiol., 2004, 54(2), pp. 420-438), and is a kind of mechanosensitive channel (EMBO J., 1999, 18(7):1730-7). As a method of imparting L-glutamic acid-producing ability to coryneform bacteria, a mutation is specifically disclosed in the present disclosure. The effect of this mutation on L-glutamic acid production has not been reported.

Accordingly, the present inventors have constructed a mutant strain in which the activity of citramalate synthase has been weakened or inactivated, and a mutant strain having an additional mutation induced in the YggB protein which is involved in glutamic acid release, and have found that the mutant strains have excellent L-glutamic acid productivity, thereby completing the present disclosure.

### [Prior Art Documents]

### [Patent Documents]

Korean Patent No. 10-1138289

### DISCLOSURE

### TECHNICAL PROBLEM

An object of the present disclosure is to provide a *Corynebacterium sp.* mutant strain in which the activity of citramalate synthase has been weakened or inactivated and which has enhanced L-glutamic acid productivity.

Another object of the present disclosure is to provide a method for producing L-glutamic acid, the method comprising steps of:
(a) culturing the *Corynebacterium sp.* mutant strain in a medium; and
(b) recovering L-glutamic acid from the cultured mutant strain or the medium.

### TECHNICAL SOLUTION

One aspect of the present disclosure provides a *Corynebacterium sp.* mutant strain in which the activity of citramalate synthase has been weakened or inactivated and which has enhanced L-glutamic acid productivity.

In the present disclosure, the term "citramalate synthase" refers to an enzyme that catalyzes the conversion of acetyl-CoA and pyruvate into citramalate and coenzyme A.

In the present disclosure, the strain having "enhanced productivity" means that L-glutamic acid productivity of the strain is higher than that of a parent strain thereof.

In the present disclosure, the term "parent strain" refers to a wild-type strain or mutant strain to be mutated, and includes a strain that is to be mutated directly or to be transformed with a recombinant vector or the like. In the present disclosure, the parent strain may be a wild-type *Corynebacterium glutamicum* strain or a strain mutated from the wild-type strain. Preferably, the parent strain may be a *Corynebacterium glutamicum* KCTC 11558BP strain.

According to one embodiment of the present disclosure, the *Corynebacterium* sp. mutant strain having enhanced L-glutamic acid productivity may have weakened or inactivated activity of citramalate synthase compared to the parent strain thereof.

In the present disclosure, the term "weakened activity" means that the enzymatic activity of citramalate synthase in the cell has decreased compared to that in a parent strain such as a wild-type strain or a strain before modification. The term "weakened" also includes: a case in which the activity of the enzyme itself has decreased compared to the activity of the enzyme in the parent microorganism due to a mutation in the gene encoding the enzyme; a case in which the overall activity of the enzyme in the cell is lower than that in the wild-type strain or the strain before modification, by a reduced expression level of due to inhibited transcription or translation of the gene encoding the enzyme; and a combination thereof. In addition, in the present disclosure, the term "inactivated" means that the gene encoding citramalate synthase is not expressed at all compared to that in the wild-type strain or the strain before modification, or that the gene has no activity even though it is expressed.

According to one embodiment of the present disclosure, weakening or inactivation of the enzymatic activity of citramalate synthase may be achieved by applying various methods well known in the art. Examples of these methods include, but are not limited to, a method of replacing the gene encoding the enzyme on the chromosome with a mutated gene so that the activity of the enzyme is decreased, including a case where the activity of the enzyme is completely removed; a method of introducing a mutation into the expression control sequence (e.g., a promoter) of the gene encoding the enzyme on the chromosome; a method of replacing the expression control sequence of the gene encoding the enzyme with a weakly active or inactive sequence; a method of deleting all or part of the gene encoding the enzyme on the chromosome; a method of introducing an antisense oligonucleotide (e.g., antisense RNA) that binds complementarily to the transcript of the gene on the chromosome and inhibits translation of the mRNA into the enzyme; a method of making the attachment of ribosomes impossible by artificially adding a sequence complementary to the SD sequence to the front end of the SD sequence of the gene encoding the enzyme to form a secondary structure; a reverse transcription engineering (RTE) method of adding a promoter to the 3' end of the open reading frame (ORF) of the corresponding sequence so as to be reverse-transcribed; and combinations thereof.

According to one embodiment of the present disclosure, citramalate synthase may be encoded by 2-isopropylmalate synthase *(leuA)* gene.

It is known that the 2-isopropylmalate synthase *(leuA)* gene that is used in the present disclosure has high homology to the citramalate synthase *(cimA)* gene and plays the role of citramalate synthase in a *Corynebacterium* sp. strain. According to one embodiment of the present disclosure, the *leuA* gene consists of the nucleotide sequence of SEQ ID NO: 14.

According to one embodiment of the present disclosure, weakening or inactivation of the activity of citramalate synthase may be weakening or inactivation of the activity of citramalate synthase due to decreased expression of the *leuA* gene encoding citramalate synthase.

In the present disclosure, the term "decreased expression" means that the expression level of the gene is decreased compared to the original expression level, and includes a case where the expression is completely inhibited, so that the expression does not occur. When gene expression in a microorganism is to be deceased, the expression may be decreased by replacing the translation initiation codon of the gene or genetically manipulating the existing gene to reduce the expression level thereof. The method of modifying the sequence to control expression may be performed by inducing mutation in the expression control sequence in the nucleic acid sequence of the expression control sequence by deletion, insertion, non-conservative or conservative substitution, or a combination thereof, or by replacing the promoter with a weaker promoter. Examples of the expression control sequence includes, but is not limited to, a promoter, an operator sequence, a sequence encoding a ribosome binding site, and a sequence for controlling termination of transcription and translation. When a gene whose expression is to be decreased is present in a microorganism to be mutated, it is possible to decrease expression of the gene, for example, by replacing a native promoter that drives expression of the gene with a weak promoter or by introducing mutation. Furthermore, it is possible to decrease expression of the gene by deleting all or part of the existing gene.

According to one embodiment of the present disclosure, decreasing the expression of the *leuA* gene may be performed by inducing mutation into the nucleic acid sequence by deletion, insertion, non-conservative or conservative substitution, or a combination thereof, or replacing the promoter with a weaker promoter.

In the *leuA* gene-deleted *Corynebacterium* sp. mutant strain according to one embodiment of the present disclosure, the production of citramalate as a by-product may decrease compared to that in the parent strain, and L-glutamic acid production may increase by saving acetyl-CoA.

According to one embodiment of the present disclosure, the *Corynebacterium* sp. mutant strain may have an additional mutation caused by substitution of alanine or valine for at least one leucine selected from the group consisting of leucine at amino acid position 93 and leucine at amino acid position 109 in the amino acid sequence of a YggB protein represented by SEQ ID NO: 15.

In the present disclosure, the *yggB* gene refers to a gene encoding a mechanosensitive channel. The *yggB* gene corresponds to a sequence complementary to a sequence of 1,336,092 to 1,337,693 in the genomic sequence registered under Genbank Accession No. NC_003450 in the NCBI database, and is also called NCg11221. The YggB protein encoded by the *yggB* gene is registered under Genbank Accession No. NP_600492. According to one embodiment of the present disclosure, the YggB protein encoded by the *yggB* gene consists of the amino acid sequence of SEQ ID NO: 15.

According to one embodiment of the present disclosure, due to the mutation caused by substitution of alanine or valine for at least one leucine selected from the group consisting of leucine at amino acid position 93 and leucine at amino acid position 109 in the amino acid sequence of the YggB protein represented by SEQ ID NO: 15, a methyl group which is involved in the pore of the YggB protein, from which L-glutamic acid may be released, is removed, so that the diameter of the pore may be increased, whereby the strain having the mutation may have enhanced L-glutamic acid productivity.

Specifically, the mutant strain may include a mutant of the YggB protein due to mutation, or may be transformed with a vector containing a polynucleotide encoding the mutant of the YggB protein.

In the present disclosure, the term "vector" refers to any vehicle for the cloning of and/or transfer of a nucleic acid into a host cell. The vector may be a replicon to which another DNA fragment may be attached so as to bring about the replication of the attached fragment. The term "Replicon" refers to any genetic element (e.g., plasmid, phage, cosmid, chromosome, virus) that functions as an autonomous unit of DNA replication *in vivo*, i.e., capable of replication under its own control. In the present disclosure, the vector is not particularly limited as long as it may replicate in a host, and any vector known in the art may be used. The vector used for the construction of the recombinant vector may be a plasmid, cosmid, virus or bacteriophage in a native state or a recombinant state. Example of a phage vector or cosmid vector that may be used include pWE15, M13, λEMBL3, λEMBL4, λFIXII, λDASHII, λZAPII, λgt10, λgt11, Charon4A, and Charon21A, and examples of a plasmid vector that may be used include a pDZ vector, and pBR-based, pUC-based, pBluescriptII-based, pGEM-based, pTZ-based, pCL-based and pET-based vectors. A vector that may be used is not particularly limited, and a known expression vector may be used, but is not limited thereto.

In the present disclosure, the term "transformation" means introducing a gene into a host cell so that the gene may be expressed in the host cell. For the transformed gene, it does not matter whether the gene is inserted into the chromosome of the host cell or located outside of the chromosome, as long as the gene may be expressed in the host cell.

The *Corynebacterium* sp. mutant strain having a deletion of the *leuA* gene and a mutation in the YggB protein according to one embodiment of the present disclosure may have increased L-glutamic acid production than the parent strain thereof and require a shortened culturing time for L-glutamic acid production, and thus may efficiently produce L-glutamic acid.

According to one embodiment of the present disclosure, the *Corynebacterium* sp. strain may be *Corynebacterium glutamicum.*

According to one embodiment of the present disclosure, the *Corynebacterium* sp. strain may be, but is not limited to, *Corynebacterium ammoniagenes, Corynebacterium acetoacidophilum, Corynebacterium acetoglutamicum, Corynebacterium alkanolyticum, Corynebacterium callunae, Corynebacterium lilium, Corynebacterium melassecola, Corynebacterium thermoaminogenes, Corynebacterium efficiens,* or *Corynebacterium herculis.*

According to one embodiment of the present disclosure, the *Corynebacterium* sp. strain is most preferably *Corynebacterium glutamicum* KCTC 11558BP.

Another aspect of the present disclosure provides a method for producing L-glutamic acid, the method comprising steps of:
(a) culturing the *Corynebacterium sp.* mutant strain in a medium; and
(b) recovering L-glutamic acid from the cultured mutant strain or the medium.

According to one embodiment of the present disclosure, the culturing may be performed using a suitable medium and culture conditions known in the art, and any person skilled in the art may easily adjust and use the medium and the culture conditions. Specifically, the medium may be a liquid medium, but is not limited thereto. Examples of the culturing method include, but are not limited to, batch culture, continuous culture, fed-batch culture, or a combination thereof.

According to one embodiment of the present disclosure, the medium should meet the requirements of a specific strain in a proper manner, and may be appropriately modified by a person skilled in the art. For the culture medium for the *Corynebacterium* sp. strain, reference may be made to, but not limited to, a known document (Manual of Methods for General Bacteriology, American Society for Bacteriology, Washington D.C., USA, 1981).

According to one embodiment of the present disclosure, the medium may contain various carbon sources, nitrogen sources, and trace element components. Examples of carbon sources that may be used include: sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol, and organic acids such as acetic acid. These substances may be used individually or as a mixture, but are not limited thereto. Examples of nitrogen sources that may be used include peptone, yeast extract, meat extract, malt extract, corn steep liquor, soybean meal, urea, or inorganic compounds such as ammonium sulfate, ammonium chloride, ammonium phosphate, ammonium carbonate, and ammonium nitrate. The nitrogen sources may also be used individually or as a mixture, but are not limited thereto. Examples of phosphorus sources that may be used include, but are not limited to, potassium dihydrogen phosphate or dipotassium hydrogen phosphate or the corresponding sodium salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be used in the culture medium. The medium or individual components may be added to the culture medium batchwise or in a continuous manner by a suitable method during culturing, but are not limited thereto.

According to one embodiment of the present disclosure, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present disclosure, in the step of recovering L-glutamic acid from the cultured mutant strain and the culture medium, the produced L-glutamic acid may be collected or recovered from the medium using a suitable method known in the art depending on the culture method. Examples of the method for collecting or recovering L-glutamic acid include, but are not limited to, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity and size exclusion).

According to one embodiment of the present disclosure, the step of recovering L-glutamic acid may be performed by centrifuging the culture medium at a low speed to remove biomass and separating the obtained supernatant through ion-exchange chromatography.

According to one embodiment of the present disclosure, the step of recovering L-glutamic acid may include a process of purifying L-glutamic acid.

### Advantageous Effects

The mutant strain according to one embodiment of the present disclosure has reduced production of citramalate as a by-product due to weakening or inactivation of the activity of citramalate synthase and has excellent L-glutamic acid productivity. In addition, the strain having an additional mutation in the YggB protein may produce L-glutamic acid in an improved yield due to enhancement of glutamic acid release. Thus, when the mutant strain is used, it is possible to more effectively produce L-glutamic acid.

### Brief Description of Drawings

FIG. 1 shows the synthetic pathway of citramalate.
FIG. 2 shows a pKLA vector constructed to disrupt the *leuA* gene in the *Corynebacterium glutamicum* KCTC 11558BP strain.
FIG. 3 shows the positions of primers 1 and 4 in a leuA gene-deleted region.
FIG. 4a shows the structure of YggB protein.
FIG. 4b shows the pore structure of the YggB protein having a substitution of alanine for leucine at position 93 in the amino acid sequence of the YggB protein.
FIG. 4c shows the pore structure of the YggB protein having a substitution of alanine for leucine at position 109 in the amino acid sequence of the YggB protein.
FIG 5. shows a pKY93 vector constructed to substitute another amino acid for the amino acid at position 93 in the amino acid sequence of the YggB protein in an I10 strain.

### Mode for Invention

Hereinafter, one or more embodiments will be described in more detail with reference to examples. However, these examples serve to illustratively describe one or more embodiments, and the scope of the present disclosure is not limited to these examples.

### Example 1. Intracellular Metabolite Analysis of Strain after Completion of Fermentation

The *Corynebacterium glutamicum* KCTC 11558BP strain, which is a mutant strain obtained by chemically treating an I6 strain with nitrosoguanidine (NTG) that causes DNA mutation, has molasses resistance, and thus may produce L-glutamic acid in high yield by using inexpensive molasses as a main carbon source (Korean Patent Application Publication No. 10-2011-0034718).

*Corynebacterium glutamicum* I6 (hereinafter referred to as 16) and *Corynebacterium glutamicum* KCTC 11558BP (hereinafter referred to as KCTC 11558BP) having the ability to produce glutamic acid were analyzed for the amount of metabolites and L-glutamic acid in the cells.

Each of KCTC 11558BP and I6 was inoculated into 25 ml of CM liquid medium (containing, per liter, 1% glucose, 1% polypeptone, 0.5% yeast extract, 0.5% beef extract, 0.25% NaCl, 0.2% urea, 50% NaOH 100 1, pH 6.8), and then cultured with shaking at 200 rpm at 30°C. When cell growth reached an exponential phase during culture, cells were separated from the culture medium by rapid vacuum filtration (Durapore HV, 0.45 m; Millipore, Billerica, MA). The filter having the cells adsorbed thereon was washed twice with 10 ml of cooling water, and then immersed in methanol containing 5 M morpholine ethanesulfonic acid and 5 M methionine sulfone for 10 minutes to stop the reaction. The extract obtained therefrom was mixed with the same amount of chloroform and a 0.4-fold volume of water, and then only the aqueous phase was applied to a spin column to remove protein contaminants. The relative amount of citramalate in the filtered extract was analyzed using capillary electrophoresis mass spectrometry, and the results are shown in Table 1 below.

In addition, L-glutamic acid production was analyzed by performing high performance liquid chromatography (HPLC; Agilent, USA) using a C18 column (Apollo C18, 5 µm, 150 mm x 4.6 mm). The results are shown in Table 1 below. An analysis buffer containing 8.85 g/L of KH₂PO₄, 1.652 g/L of tetrabutyl ammonium hydroxide solution, 10 ml/L of acetonitrile and 12.5 mg/L of sodium azide and having a pH of 3.15 (adjusted with phosphoric acid) was used after filtration through a 0.45 µm membrane-filter (HA).

As shown in Table 1 below, it was confirmed that the amount of citramalate produced by KCTC 11558BP increased by about 3 times compared to that produced by the control 16.

**[Table 1]**

| | I6 (control) | KCTC 11558BP |
|---|---|---|
| L-glutamic acid production (g/L) | 34 | 37.5 |
| Citramalate (relative amount) | 130.2 | 330.1 |

### Example 2. Construction of leuA Gene-Deleted Corynebacterium glutamicum Mutant Strain

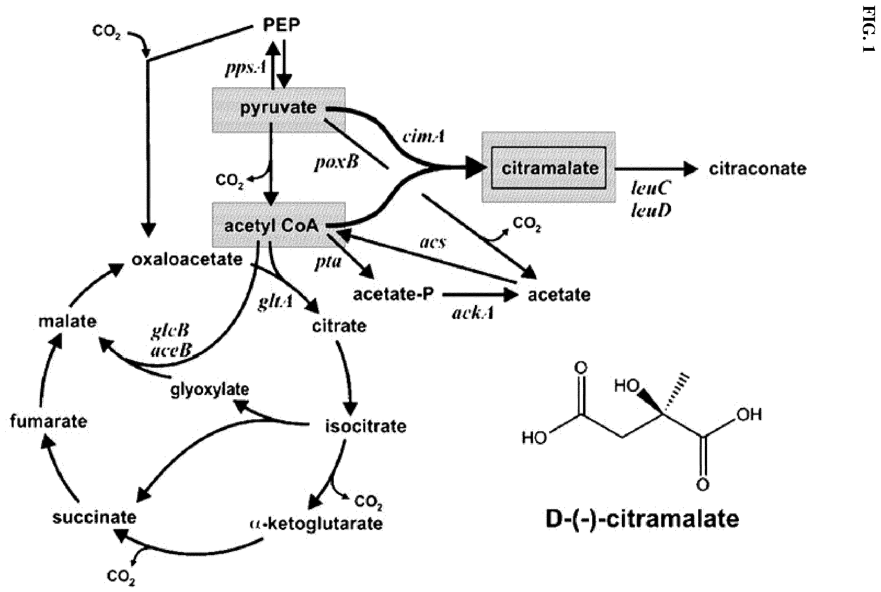

FIG. 1 shows the synthetic pathway of citramalate. Citramalate is produced by the citramalate synthase *(cimA)* gene in *E. coli,* but the *cimA* gene does not exist in *Corynebacterium glutamicum.* However, since it is known that the *leuA* gene having high homology to the *cimA* gene plays the role of citramalate synthase in *Corynebacterium glutamicum,* the present inventors have attempted to reduce the production of citramalate as a by-product by disrupting 2-isopropylmalate synthase *(leuA)* and save the substrate acetyl-CoA which is used in the production of L-glutamic acid, thereby further increasing L-glutamic acid productivity. The nucleotide sequence of the *leuA* gene is set forth in SEQ ID NO: 14.

### Example 2-1. Construction of Vector for Disruption of leuA Gene

Corynebacterium glutamicum KCTC 11558BP having L-glutamic acid-producing ability was used for disruption of the leuA gene (NCg10245) in *Corynebacterium glutamicum.*

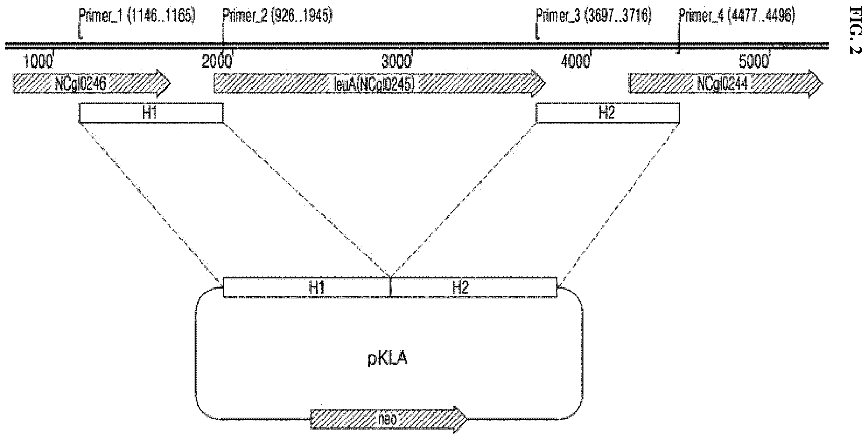

The chromosomal DNA of the *Corynebacterium glutamicum* KCTC 11558BP strain was isolated. Using the isolated DNA as a template, PCR was performed using each of a set of primers 1 and 2 and a set of primers 3 and 4 for 30 cycles, each consisting of 95°C for 30 sec, 57°C for 30 sec, and 72°C for 60 sec. The obtained PCR products (H1 and H2 in FIG. 2) were purified.

The purified PCR products were then used as a template for crossover polymerase chain reaction (PCR) and amplified again using a set of primers 1 and 4.

The 1600-bp crossover PCR product for deleting the *leuA* gene was purified, digested with *Xba*I and *Sal*I restriction enzymes (Takara, Japan), and then cloned into a pK19mobSacB vector (Gene, 145: 69-73, 1994) digested with the same restriction enzymes, thereby constructing a vector for disruption of the *leuA* gene. The constructed vector was named pKLA. FIG. 2 shows the pKLA vector constructed to disrupt the *leuA* gene in the *Corynebacterium glutamicum* KCTC 11558BP strain.

**[Table 2]**

| Primer name | Sequence (5'→3') | SEQ ID NO |
|---|---|---|
| Primer 1 | 5'-TCTAGACCCTTCCCGGAAACCCCCAC-3' | SEQ ID NO: 1 |
| Primer 2 | 5'-GGGGTTTCGATCTTGGCAGG-3' | SEQ ID NO: 2 |
| Primer 3 | 5'-ACCGCGCGCTGGACGTCAAC-3' | SEQ ID NO: 3 |
| Primer 4 | 5'-GTCGACTGGCCGAAACGCTTAGCGCT-3' | SEQ ID NO: 4 |

### Example 2-2. Transformation of Corynebacterium glutamicum Strain and Construction of leuA-Deleted Mutant Strain

Using the method of van der Rest (Appl. Microbiol. Biotechnol., 52, 541-545, 1999), the vector pKLA constructed in Example 2-1 was introduced by electroporation into the *Corynebacterium glutamicum* KCTC 11558BP strain (hereinafter referred to as KCTC 11558BP) made electrocompetent by an electrocompetent cell preparation.

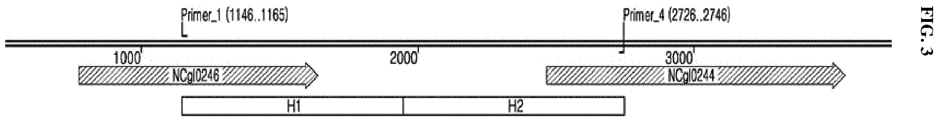

Specifically, the KCTC 11558BP strain was first cultured in 100 ml of a 2YT medium (16 g/l tryptone, 10 g/l yeast extract, 5 g/l sodium chloride) supplemented with 2% glucose, and 1 mg/ml of isonicotinic acid hydrazine and 2.5% glycine were added to the same medium free of glucose. Then, the seed culture medium was inoculated to reach an OD₆₁₀ value of 0.3, and then cultured for at 18°C and 180 rpm for 12 to 16 hours until the OD₆₁₀ value reached 1.2 to 1.4. After keeping on ice for 30 minutes, centrifugation was performed at 4,000 rpm at 4°C for 15 minutes. Thereafter, the supernatant was discarded and the precipitated KCTC 11558BP strain was washed 4 times with a 10% glycerol solution and finally re-suspended in 0.5 ml of a 10% glycerol solution. Electroporation was performed using a Bio-Rad electroporator. The competent cells prepared by the above-described method were placed in an electroporation cuvette (0.2 mm), and the pKLA vector constructed in Example 2-1 was added thereto, followed by electroporation under the conditions of 2.5 kV, 200 Ω and 12.5 µF. Immediately after completion of the electroporation, 1 ml of a regeneration medium (18.5 g/l brain heart infusion, 0.5 M sorbitol) was added to the cells which were then heat-treated at 46°C for 6 minutes. Next, the cells were cooled at room temperature, transferred into a 15-ml cap tube, incubated at 30°C for 2 hours, and plated on a selection medium (5 g/l tryptone, 5 g/l NaCl, 2.5 g/l yeast extract, 18.5 g/l brain heart infusion powder, 15 g/l agar, 91 g/l sorbitol, 20 µg/l kanamycin). The cells were cultured at 30°C for 72 hours, and the generated colonies were cultured in BHI medium until a stationary phase to induce secondary recombination. Then, the cells were diluted to 10⁻⁵ to 10⁻⁷ cells, and plated on an antibiotic-free plate (containing 10% sucrose), and a strain having no kanamycin resistance and grown in the medium containing 10% sucrose was selected. The selected strain was confirmed to be an *leuA* gene-deleted *Corynebacterium glutamicum* strain using a set of primers 1 and 4 described in Table 2 above, and the *leuA* gene-deleted strain was name "I10". FIG. 3 shows the positions of primers 1 and 4 in the leuA gene-deleted region.

### Example 2-3. Confirmation of Auxotrophy of leuA―Deleted Strain (110)

The leuA-deleted strain (hereinafter referred to as I10) constructed in Example 2-2 becomes a leucine auxotrophic strain due to deletion of the *leuA* gene. To confirm this fact, the strain was plated in each of a minimal medium prepared to have the composition shown in Table 3 below and the minimal medium supplemented with leucine and then was cultured at 30°C for 24 hours, and the degree of growth thereof was measured. The results are shown in Table 4 below.

**[Table 3]**

| Composition of minimal medium | |
|---|---|
| Glucose | 20 g/L |
| MgSO₄. 7H₂O | 0.5 g/L |
| FeSO₄. 7H₂O | 20 mg/L |
| MnSO₄. 5H₂O | 20 mg/L |
| Urea | 2.5 g/L |
| (NH₄)₂SO₄ | 5 g/L |
| KH₂PO₄ | 1.5 g/L |
| K₂HPO₄ | 1.5 g/L |
| Thiamine-HCl | 200 µg/L |
| Biotin | 200 µg/L |
| Nicotinic acid | 200 µg/L |
| Vitamin B2 | 200 µg/L |
| Cyanocobalamine | 100 µg/L |

As shown in Table 4 below, the growth of the I10 strain decreased rapidly compared to the growth of the parent strain *Corynebacterium glutamicum* KCTC 11558BP strain in the minimal medium not supplemented with leucine, and was restored in the minimal medium supplemented with leucine. Thus, the deletion of the *leuA* gene in the I10 strain was confirmed.

**[Table 4]**

| | KCTC 11558BP | I10 |
|---|---|---|
| Minimal medium | ++++ | + |
| Minimal medium + 50 ppm leucine | ++++ | ++++ |

### Example 3. Analysis of L-glutamic Acid and Citramalate Production in leuA Gene-Deleted Corynebacterium glutamicum Strain (I10)

Using an active plate medium prepared to have the composition shown in Table 5 below, each of the leuA-deleted strain (hereinafter referred to as I10) and *Corynebacterium glutamicum* KCTC 11558BP (hereinafter referred to as KCTC 11558BP) was cultured at 30°C for 24 hours. Thereafter, 10 ml of an L-glutamic acid flask medium prepared to have the composition shown in Table 6 below was placed in a 100-ml flask and inoculated with a loop of each of the cultured strains which were then cultured at 30°C and 200 rpm for 48 hours. After completion of culture, the cultures were used to perform the analysis of L-glutamic acid production and intracellular metabolites. The analysis of L-glutamic acid production and relative citramalate amount was performed in the same manner as described in Example 1.

Table 7 below shows the L-glutamic acid production and relative citramalate amount in each of I10 and KCTC 11558BP. As shown in Table 7, it could be confirmed that, in the I10 strain, the L-glutamic acid production increased and the relative citramalate amount significantly decreased.

**[Table 5]**

| **Composition of active plate medium** | |
|---|---|
| Glucose | 5 g/L |
| Yeast extract | 10 g/L |
| Urea | 3 g/L |
| KH₂PO₄ | 1 g/L |
| Biotin | 2 µ/L |
| Soybean hydrolyzate | 0.1% v/v |
| Leucine | 50 mg/L |
| Agar | 20 g/L |
| pH 7.5 | |

**[Table 6]**

| **Composition of L-glutamic acid flask medium** | |
|---|---|
| Glucose | 70 g/L |
| MgSO₄. 7H₂O | 0.4 g/L |
| Urea | 2 g/L |
| KH₂PO₄ | 1 g/L |
| Soybean hydrolyzate | 1.5% v/v |
| (NH₄)₂SO₄ | 5 g/L |
| FeSO₄. 7H₂O | 10 mg/L |
| MnSO₄. 5H₂O | 10 mg/L |
| Thiamine-HCl | 200 µg/L |
| Biotin | 2 µg/L |
| Calcium carbonate | 50 g/L |
| pH 7.6 | |

**[Table 7]**

| | **KCTC 11558BP** | **I10** |
|---|---|---|
| **L-glutamic acid production (g/L)** | 37.5 | 39.7 |
| **Citramalate (relative amount)** | 320.4 | 8.5 |

### Example 4. Construction of YggB-Mutated Strain

Since it was confirmed from the results of metabolite analysis of the *leuA*-deleted strain (I10) that a lot of L-glutamic acid was accumulated inside the cells, the present inventors attempted to enhance the release of L-glutamic acid.

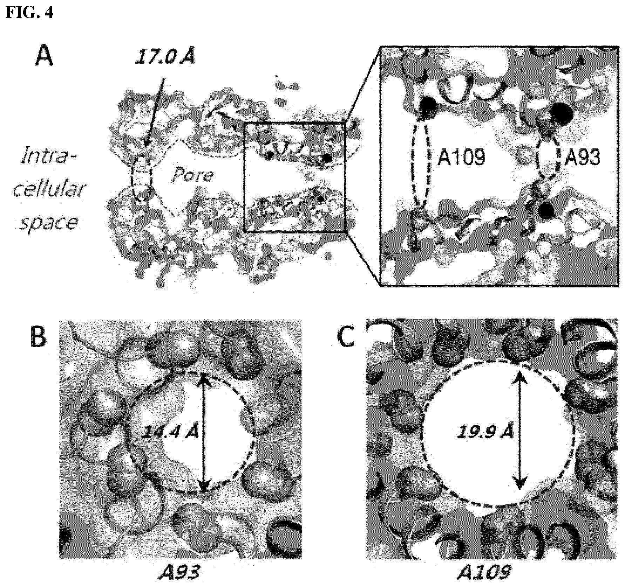

It is known that YggB is a mechanosensitive channel homolog and plays an important role in the release of L-glutamic acid in *Corynebacterium glutamicum.* The amino acid sequence of YggB protein is set forth in SEQ ID NO: 15. FIG. 4a shows the structure of the YggB protein. The structure of the YggB protein was analyzed to find a mutation capable of enhancing the release of L-glutamic acid.

FIG. 4b shows the pore structure of the YggB protein having a substitution of alanine for leucine at position 93 in the amino acid sequence of the YggB protein, and FIG. 4c shows the pore structure of the YggB protein having a substitution of alanine for leucine at position 109 in the amino acid sequence of the YggB protein.

### Example 4-1. Construction of Strain Having Substitution of Amino Acid at Position 93 in Amino Acid Sequence of YggB Protein

As shown in FIG. 4b, it was assumed that, when leucine at position 93 in the amino acid sequence of the YggB protein was substituted with alanine in order to enlarge the L-glutamic acid channel, the methyl group involved in the narrow portion inside the pore would be removed, so that the diameter of the pore would increase. Based on this assumption, a mutant strain was constructed.

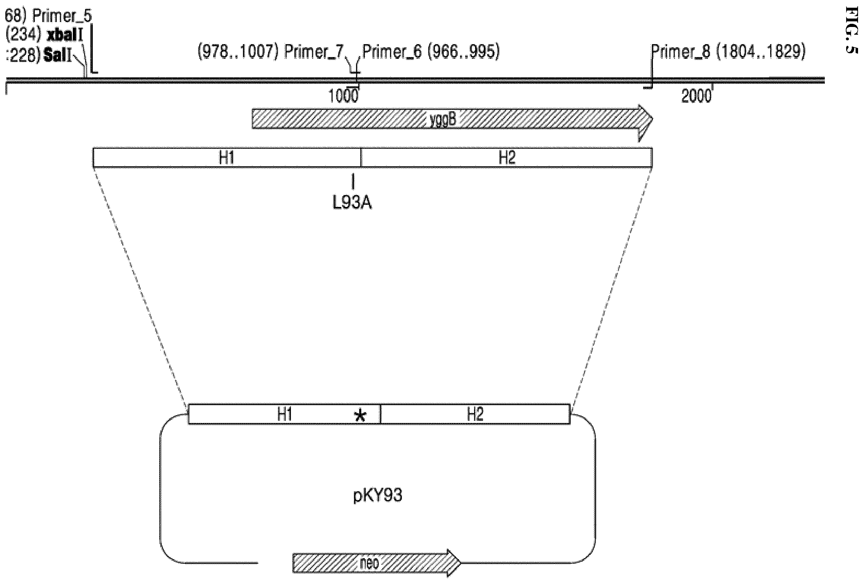

The chromosomal DNA of the I10 strain constructed in Example 2-2 was isolated. Using the isolated DNA as a template, PCR was performed using each of a set of primers 5 and 6 and a set of primers 7 and 8 for 30 cycles, each consisting of 95°C for 30 sec, 57°C for 30 sec, and 72°C for 60 sec. The obtained PCR products were purified. The purified PCR products were used as a template and amplified by crossover PCR using primers 5 and 8. The amplified product was sequenced to confirm mutation, and then digested with *Xba*I and *Sal*I restriction enzymes (Takara, Japan). A pK19mobSacB vector was digested with the same restriction enzymes and ligated with the amplified crossover PCR product, thereby constructing a vector. The constructed vector was named pKY93. The primer sequences used are shown in Table 8 below. FIG. 5 shows the pKY93 vector for substituting the amino acid at position 93 in the amino acid sequence of the YggB protein in the I10 strain.

**[Table 8]**

| **Primer** | **Sequence (5'→3')** | **SEQ ID NO** |
|---|---|---|
| Primer 5 | 5'-TCTAGATTGAGAAGCTGCCACATTCAC-3' | SEQ ID NO: 5 |
| Primer 6 | 5'-GCAGCGCCCGCGGCAGAGAAACCAAAAGCC-3' | SEQ ID NO: 6 |
| Primer 7 | 5'-CTCTGCCGCGGGCGCTGCGATTCCGGCAAC-3' | SEQ ID NO: 7 |
| Primer 8 | 5'-GTCGACGATCTGGTTTTGCTGTTTCTTCCGG-3' | SEQ ID NO: 8 |
| Primer 9 | 5'-GACTGCGCACCAGCACCAATGGCAGCTGAC-3' | SEQ ID NO: 9 |
| Primer 10 | 5'-GGTGCTGGTGCGCAGTCGATTGTTGCGGAC-3' | SEQ ID NO: 10 |
| Primer 11 | 5'-TCTAGAGGAATCAGGATTCTCACAAAGTTCAGGC-3' | SEQ ID NO: 11 |
| Primer 12 | 5'-CGGAATCGCAGTGCCCGCGAGAGAGAAACC-3' | SEQ ID NO: 12 |
| Primer 13 | 5'-CGCGGGCACTGCGATTCCGGCAACCATTGC-3' | SEQ ID NO: 13 |

In the same manner as in Example 2-2, the pKY93 vector was introduced into the I10 strain, and the transformed strain was selected and named I10-93. The I10-93 mutant strain had a substitution of alanine for leucine at amino acid position 93 in the YggB protein.

### Example 4-2. Construction of Strain Having Substitution of Amino Acid at Position 109 in Amino Acid Sequence of YggB Protein

As shown in FIG. 4c, it was assumed that, when leucine at position 109 in the amino acid sequence of the YggB protein was substituted with alanine in order to enlarge the L-glutamic acid channel, the methyl group involved in the narrow portion inside the pore would be removed, so that the diameter of the pore would increase. Based on this assumption, a mutant strain was constructed.

The chromosomal DNA of the I10 strain constructed in Example 2-2 was isolated. Using the isolated DNA as a template, PCR was performed using each of a set of primers 5 and 9 and a set of primers 10 and 8 for 30 cycles, each consisting of 95°C for 30 sec, 57°C for 30 sec, and 72°C for 60 sec. The obtained PCR products were purified. The purified PCR products were used as a template and amplified by crossover PCR using primers 5 and 8. The amplified product was sequenced to confirm mutation, and then digested with *Xba*I and *Sal*I restriction enzymes (Takara, Japan). A pK19mobSacB vector was digested with the same restriction enzymes and ligated with the amplified crossover PCR product, thereby constructing a vector. The constructed vector was named pKY109. The primer sequences used are shown in Table 8 above.

In the same manner as in Example 2-2, the pKY109 vector was introduced into the I10 strain, and the transformed strain was selected and named I10-109. The I10-109 mutant strain had a substitution of alanine for leucine at amino acid position 109 in the YggB protein.

### Example 5. Confirmation of L-glutamic Acid Productivities of Mutant Strains

The amount of L-glutamic acid produced by each of the I10, I10-93 and I10-109 strains at the flask scale and the time required for production of L-glutamic acid were measured in the same manner as in Examples 1 and 3, and the results are shown in Table 9 below.

**[Table 9]**

| | I10 | I10-93 | I10-109 |
|---|---|---|---|
| L-glutamic acid production (g/L) | 39 | 42.5 | 45.2 |
| Time (hr) | 48 | 48 | 44 |

As shown in Table 9 above, the L-glutamic acid production in each of the I10-93 and I10-109 mutant strains in which enlargement of the pore of the YggB protein was induced increased compared to that in the I10 strain. In particular, in the case of the I10-109 strain, the L-glutamic acid production significantly increased compared to that in the I10 strain, and the fermentation time was also shortened from 48 hours to 44 hours, indicating that the I10-109 strain may efficiently produce L-glutamic acid.

## Claims

1. A *Corynebacterium sp.* mutant strain in which an activity of citramalate synthase has been weakened or inactivated and which has enhanced L-glutamic acid productivity.

2. The *Corynebacterium sp.* mutant strain of claim 1, wherein the citramalate synthase is encoded by 2-isopropylmalate synthase *(leuA)* gene.

3. The *Corynebacterium sp.* mutant strain of claim 1, which has an additional mutation caused by substitution of alanine or valine for at least one leucine selected from the group consisting of leucine at amino acid position 93 and leucine at amino acid position 109 in the amino acid sequence of a YggB protein represented by SEQ ID NO: 15.

4. The *Corynebacterium sp.* mutant strain of claim 1, wherein the strain is *Corynebacterium glutamicum.*

5. A method for producing L-glutamic acid, the method comprising steps of:
(a) culturing the *Corynebacterium sp.* mutant strain according to claim 1 in a medium; and
(b) recovering L-glutamic acid from the cultured mutant strain or the medium.


<110>     DAESANG CORPORATION

<120>     Mutant strain with enhanced L-glutamic acid productivity and
          method for preparing L-glutamic acid using the same

<130>     PX190056PCT

<150>     KR 10-2018-0161190
<151>     2018-12-13

<160>     15

<170>     KoPatentIn 3.0

<210>     1
<211>     26
<212>     DNA
<213>     Artificial Sequence

<220>
<223>     primer 1


<400>     1
tctagaccct tcccggaaac ccccac                                          26


<210>     2
<211>     20
<212>     DNA
<213>     Artificial Sequence

<220>
<223>     primer 2


<400>     2
ggggtttcga tcttggcagg                                                 20


<210>     3
<211>     20
<212>     DNA
<213>     Artificial Sequence

<220>
<223>     primer 3


<400>     3
accgcgcgct ggacgtcaac                                                 20


<210>     4
<211>     26
<212>     DNA
<213>     Artificial Sequence

<220>
<223>     primer 4


<400>     4

gtcgactggc cgaaacgctt agcgct                                                26

<210>    5
<211>    27
<212>    DNA
<213>    Artificial Sequence

<220>
<223>    primer 5

<400>    5
tctagattga gaagctgcca cattcac                                               27

<210>    6
<211>    30
<212>    DNA
<213>    Artificial Sequence

<220>
<223>    primer 6

<400>    6
gcagcgcccg cggcagagaa accaaaagcc                                            30

<210>    7
<211>    30
<212>    DNA
<213>    Artificial Sequence

<220>
<223>    primer 7

<400>    7
ctctgccgcg ggcgctgcga ttccggcaac                                            30

<210>    8
<211>    31
<212>    DNA
<213>    Artificial Sequence

<220>
<223>    primer 8

<400>    8
gtcgacgatc tggttttgct gtttcttccg g                                          31

<210>    9
<211>    30
<212>    DNA
<213>    Artificial Sequence

<220>
<223>    primer 9

<400>    9
gactgcgcac cagcaccaat ggcagctgac                                        30


<210>    10
<211>    30
<212>    DNA
<213>    Artificial Sequence

<220>
<223>    primer 10


<400>    10
ggtgctggtg cgcagtcgat tgttgcggac                                        30


<210>    11
<211>    34
<212>    DNA
<213>    Artificial Sequence

<220>
<223>    primer 11


<400>    11
tctagaggaa tcaggattct cacaaagttc aggc                                   34


<210>    12
<211>    30
<212>    DNA
<213>    Artificial Sequence

<220>
<223>    primer 12


<400>    12
cggaatcgca gtgcccgcga gagagaaacc                                        30


<210>    13
<211>    30
<212>    DNA
<213>    Artificial Sequence

<220>
<223>    primer 13


<400>    13
cgcgggcact gcgattccgg caaccattgc                                        30


<210>    14
<211>    1851
<212>    DNA
<213>    Corynebacterium sp.

<400>    14

atgtctccta acgatgcatt catctccgca cctgccaaga tcgaaacccc agttgggcct 60

cgcaacgaag gccagccagc atggaataag cagcgtggct cctcaatgcc agttaaccgc 120

tacatgcctt tcgaggttga ggtagaagat atttctctgc cggaccgcac ttggccagat 180

aaaaaaatca ccgttgcacc tcagtggtgt gctgttgacc tgcgtgacgg caaccaggct 240

ctgattgatc cgatgtctcc tgagcgtaag cgccgcatgt ttgagctgct ggttcagatg 300

ggcttcaaag aaatcgaggt cggtttccct tcagcttccc agactgattt tgatttcgtt 360

cgtgagatca tcgaaaaggg catgatccct gacgatgtca ccattcaggt tctggttcag 420

gctcgtgagc acctgattcg ccgtactttt gaagcttgcg aaggcgcaaa aaacgttatc 480

gtgcacttct acaactccac ctccatcctg cagcgcaacg tggtgttccg catggacaag 540

gtgcaggtga agaagctggc taccgatgcc gctgaactaa tcaagaccat cgctcaggat 600

tacccagaca ccaactggcg ctggcagtac tcccctgagt ccttcaccgg cactgaggtt 660

gagtacgcca aggaagttgt ggacgcagtt gttgaggtca tggatccaac tcctgagaac 720

ccaatgatca tcaacctgcc ttccaccgtt gagatgatca cccctaacgt ttacgcagac 780

tccattgaat ggatgcaccg caatctaaac cgtcgtgatt ccattatcct gtccctgcac 840

ccgcacaatg accgtggcac cggcgttggc gcagctgagc tgggctacat ggctggcgct 900

gaccgcatcg aaggctgcct gttcggcaac ggcgagcgca ccggcaacgt ctgcctggtc 960

accctggcac tgaacatgct gacccagggc gttgaccctc agctggactt caccgatata 1020

cgccagatcc gcagcaccgt tgaatactgc aaccagctgc gcgttcctga gcgccaccca 1080

tacggcggtg acctggtctt caccgctttc tccggttccc accaggacgc tgtgaacaag 1140

ggtctggacg ccatggctgc caaggttcag ccaggtgcta gctccactga agtttcttgg 1200

gagcagctgc gcgacaccga atgggaggtt ccttacctgc ctatcgatcc aaaggatgtc 1260

ggtcgcgact acgaggctgt tatccgcgtg aactcccagt ccggcaaggg cggcgttgct 1320

tacatcatga agaccgatca cggtctgcag atccctcgct ccatgcaggt tgagttctcc 1380

accgttgtcc agaacgtcac cgacgctgag ggcggcgagg tcaactccaa ggcaatgtgg 1440

gatatcttcg ccaccgagta cctggagcgc accgcaccag ttgagcagat cgcgctgcgc 1500

gtcgagaacg ctcagaccga aaacgaggat gcatccatca ccgccgagct catccacaac 1560

ggcaaggacg tcaccgtcga tggccgcggc aacggcccac tggccgctta cgccaacgcg 1620

ctggagaagc tgggcatcga cgttgagatc caggaataca accagcacgc ccgcacctcg 1680

ggcgacgatg cagaagcagc cgcctacgtg ctggctgagg tcaacggccg caaggtctgg 1740

ggcgtcggca tcgctggctc catcacctac gcttcgctga aggcagtgac ctccgccgta 1800

aaccgcgcgc tggacgtcaa ccacgaggca gtcctggctg gcggcgttta a 1851

<210>    15
<211>    533
<212>    PRT
<213>    Corynebacterium sp.

<400>    15
Met Ile Leu Gly Val Pro Ile Gln Tyr Leu Leu Tyr Ser Leu Trp Asn
1               5                   10                  15

Trp Ile Val Asp Thr Gly Phe Asp Val Ala Ile Ile Leu Val Leu Ala
            20                  25                  30

Phe Leu Ile Pro Arg Ile Gly Arg Leu Ala Met Arg Ile Ile Lys Arg
        35                  40                  45

Arg Val Glu Ser Ala Ala Asp Ala Asp Thr Thr Lys Asn Gln Leu Ala
    50                  55                  60

Phe Ala Gly Val Gly Val Tyr Ile Ala Gln Ile Val Ala Phe Phe Met
65                  70                  75                  80

Leu Ala Val Ser Ala Met Gln Ala Phe Gly Phe Ser Leu Ala Gly Ala
                85                  90                  95

Ala Ile Pro Ala Thr Ile Ala Ser Ala Ala Ile Gly Leu Gly Ala Gln
            100                 105                 110

Ser Ile Val Ala Asp Phe Leu Ala Gly Phe Phe Ile Leu Thr Glu Lys
        115                 120                 125

Gln Phe Gly Val Gly Asp Trp Val Arg Phe Glu Gly Asn Gly Ile Val
    130                 135                 140

Val Glu Gly Thr Val Ile Glu Ile Thr Met Arg Ala Thr Lys Ile Arg
145                 150                 155                 160

Thr Ile Ala Gln Glu Thr Val Ile Ile Pro Asn Ser Thr Ala Lys Val
                165                 170                 175

Cys Ile Asn Asn Ser Asn Asn Trp Ser Arg Ala Val Val Val Ile Pro
            180                 185                 190

Ile Pro Met Leu Gly Ser Glu Asn Ile Thr Asp Val Ile Ala Arg Ser
        195                 200                 205

Glu Ala Ala Thr Arg Arg Ala Leu Gly Gln Glu Lys Ile Ala Pro Glu
    210                 215                 220

Ile Leu Gly Glu Leu Asp Val His Pro Ala Thr Glu Val Thr Pro Pro
225                 230                 235                 240

Thr Val Val Gly Met Pro Trp Met Val Thr Met Arg Phe Leu Val Gln
                245                 250                 255

Val Thr Ala Gly Asn Gln Trp Leu Val Glu Arg Ala Ile Arg Thr Glu
            260                 265                 270

Ile Ile Ser Glu Phe Trp Glu Glu Tyr Gly Ser Ala Thr Thr Thr Ser
        275                 280                 285

Gly Thr Leu Ile Asp Ser Leu His Val Glu His Glu Glu Pro Lys Thr
    290                 295                 300

Ser Leu Ile Asp Ala Ser Pro Gln Ala Leu Lys Glu Pro Lys Pro Glu
305                 310                 315                 320

Ala Ala Ala Thr Val Ala Ser Leu Ala Ala Ser Ser Asn Asp Asp Ala
                325                 330                 335

Asp Asn Ala Asp Ala Ser Val Ile Asn Ala Gly Asn Pro Glu Lys Glu
            340                 345                 350

Leu Asp Ser Asp Val Leu Glu Gln Glu Leu Ser Ser Glu Glu Pro Glu
        355                 360                 365

Glu Thr Ala Lys Pro Asp His Ser Leu Arg Gly Phe Phe Arg Thr Asp
    370                 375                 380

Tyr Tyr Pro Asn Arg Trp Gln Lys Ile Leu Ser Phe Gly Gly Arg Val
385                 390                 395                 400

Arg Met Ser Thr Ser Leu Leu Leu Gly Ala Leu Leu Leu Leu Ser Leu
                405                 410                 415

Phe Lys Val Met Thr Val Glu Pro Ser Glu Asn Trp Gln Asn Ser Ser
            420                 425                 430

Gly Trp Leu Ser Pro Ser Thr Ala Thr Ser Thr Ala Val Thr Thr Ser
        435                 440                 445

Glu Thr Ser Ala Pro Val Ser Thr Pro Ser Met Thr Val Pro Thr Thr
    450                 455                 460

Val Glu Glu Thr Pro Thr Met Glu Ser Asn Val Glu Thr Gln Gln Glu
465                 470                 475                 480

Thr Ser Thr Pro Ala Thr Ala Thr Pro Gln Arg Ala Asp Thr Ile Glu
                485                 490                 495

Pro Thr Glu Glu Ala Thr Ser Gln Glu Glu Thr Thr Ala Ser Gln Thr
            500                 505                 510

Gln Ser Pro Ala Val Glu Ala Pro Thr Ala Val Gln Glu Thr Val Ala
        515                 520                 525

Pro Thr Ser Thr Pro
    530